(19) Europäisches Patentamt
European Patent Office
Office européen des brevets

(11) Veröffentlichungsnummer: **0 048 298**
**A1**

(12) # EUROPÄISCHE PATENTANMELDUNG

(21) Anmeldenummer: **80105712.6**

(22) Anmeldetag: **23.09.80**

(51) Int. Cl.[3]: **A 61 K 31/505**
A 61 K 31/63, A 61 K 31/635

(43) Veröffentlichungstag der Anmeldung:
**31.03.82 Patentblatt 82/13**

(84) Benannte Vertragsstaaten:
**AT BE CH DE FR GB IT LI NL SE**

(71) Anmelder: **LUDWIG HEUMANN & CO GMBH**
**Heideloffstrasse 18-28**
**D-8500 Nürnberg(DE)**

(72) Erfinder: **Haake, Manfred, Prof. Dr., Prof.**
**Goldbergstrasse 19**
**D-3550 Marburg(DE)**

(72) Erfinder: **Ahrens, Kurt Henning, Dr.**
**Praterstrasse 9**
**D-8500 Nürnberg(DE)**

(74) Vertreter: **Kraus, Walter, Dr. et al,**
**Patentanwälte Dres. Kraus & Weisert Irmgardstrasse 15**
**D-8000 München 71(DE)**

(54) **Verwendung von Embonaten von 2,4-Diamino-benzylpyrimidinen.**

(57) Die Erfindung betrifft die Verwendung von Embonaten von 2,4-Diamino-benzylpyrimidinen der allgemeinen Formel

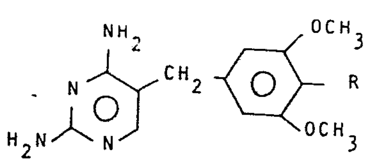

. 1/2

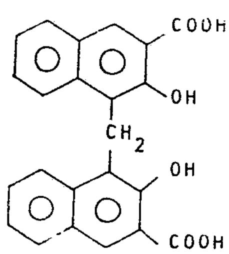

in der R für Methoxy oder Methoxyäthoxy steht, in mikronisierter Form zur Herstellung von peroralen flüssigen Darreichungsformen, die 2,4-Diamino-benzylpyrimidin gegebenenfalls zusammen mit einem Sulfonamid enthalten. Die so erhaltenen flüssigen Darreichungsformen zeichnen sich auch ohne Zusatz eines Geschmackskorrigens durch einen angenehmen nicht-bitteren Geschmack aus.

EP 0 048 298 A1

0048298

2645 WK/rm

LUDWIG HEUMANN & CO GMBH, 8500 Nürnberg

# Verwendung von Embonaten von 2,4-Diamino-benzylpyrimidinen

Die Erfindung betrifft die Verwendung von Embonaten von bestimmten 2,4-Diamino-benzylpyrimidinen in mikronisierter Form zur Herstellung von peroralen flüssigen Darreichungsformen, vorzugsweise von Säften, die insbesondere in der Kinderheilkunde verwendet werden. Diese flüssigen Darreichungsformen können gegebenenfalls noch ein Sulfonamid enthalten, wie es üblicherweise in Kombinationspräparaten mit 2,4-Diamino-benzylpyrimidinen verwendet wird.

2,4-Diamino-benzylpyrimidine sind wegen ihrer antibakteriellen Wirkung wertvolle Arzneimittel. Insbesondere das in der DE-PS 943 706 beschriebene Trimethoprim (2,4-Diamino-5-(3',4',5'-trimethoxybenzyl)-pyrimidin) und das in der DE-PS 2 313 361 beschriebene Tetroxoprim (2,4-Diamino-5-(3',5'-dimethoxy-4'-methoxyäthoxy-benzyl)-pyrimidin) haben sich in der Praxis als Chemotherapeutika bewährt. Handelspräparate dieser Substanzen enthalten neben dem 2,4-Diamino-benzylpyrimidin in Form der freien Base noch ein übliches Sulfonamid, wie z.B. Sulfadiazin.

Bei Trimethoprim und Tetroxoprim hat sich jedoch in der Vergangenheit der bittere Eigengeschmack der Substanzen als Schwierigkeit bei der Formulierung von flüssigen Zubereitungen, wie z.B. Säften, erwiesen. Bei bekannten flüssigen Zubereitungsformen von 2,4-Diamino-benzylpyrimidinen hat man bislang versucht, den bitteren Eigengeschmack durch die Verwendung von intensiv schmeckenden und stark duftenden Geschmackskorrigenzien zu überdecken.

Der Erfindung liegt daher die Aufgabe zugrunde, die Nachteile der bekannten flüssigen Zubereitungsformen zu überwinden und perorale flüssige Darreichungsformen, insbesondere Säfte, von 2,4-Diamino-benzylpyrimidinen zur Verfügung zu stellen, die auch ohne Verwendung von Geschmackskorrigenzien keinen bitteren Geschmack aufweisen.

Diese Aufgabe wird erfindungsgemäß durch Verwendung von Embonaten von 2,4-Diamino-benzylpyrimidinen der allgemeinen Formel:

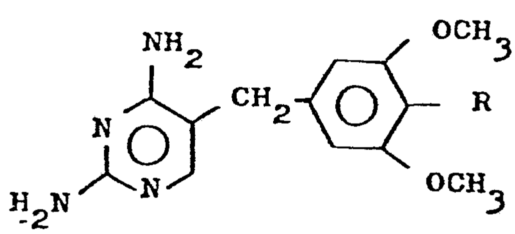

. 1/2

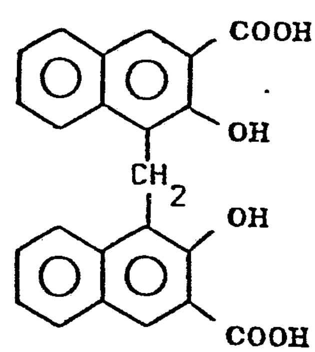

in mikronisierter Form zur Herstellung von peroralen flüssigen Darreichungsformen, die 2,4-Diamino-benzylpyrimidine, gegebenenfalls zusammen mit einem Sulfonamid, enthalten, gelöst.

Säureadditionssalze von Trimethoprim mit pharmazeutisch verträglichen Säuren sind beispielsweise aus der DE-OS 2 461 570 bekannt, doch sind die darin beschriebenen pharmazeutischen Formulierungen ausschließlich für die Augen- und/oder Ohrbehandlung vorgesehen. Bei diesen bekannten pharmazeutischen Formulierungen handelt es sich ausschließlich um topische Anwendungsformen. Demgegenüber werden erfindungsgemäß die vorgenannten Embonate für flüssige perorale Darreichungsformen, insbesondere Suspensionssäfte, verwendet.

Die oben beschriebenen Embonate sind bislang in der Literatur nicht beschrieben worden, d.h. es handelt sich um neue Verbindungen.

Die Herstellung der Embonate erfolgt in einfacher Weise durch Umsetzung des 2,4-Diamino-benzylpyrimidins mit der entsprechenden Menge Embonsäure bzw. deren Alkalisalz in einem geeigneten Lösungsmittel, vorzugsweise bei erhöhter Temperatur bzw. Siedetemperatur. Als Lösungsmittel kommen z.B. Wasser, Alkohole bzw. Gemische aus Alkoholen, wie Isopropanol, und Ketone, wie Aceton, in Betracht. Durch Erkaltenlassen der Reaktionslösung kristallisiert das gewünschte Embonat aus, welches gefiltert, gewaschen und getrocknet wird.

Somit werden gemäß der Erfindung auch neue Embonate von 2,4-Diamino-benzylpyrimidinen der allgemeinen Formel

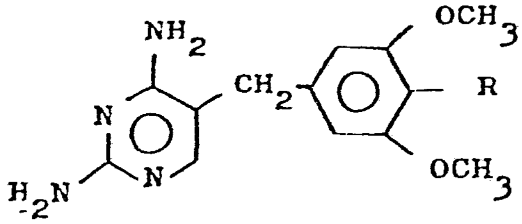 . 1/2 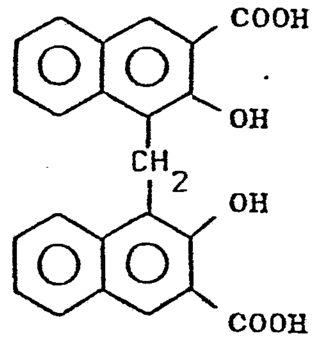

in der R für Methoxy oder Methoxyäthoxy steht, in Betracht gezogen. Durch die Erfindung werden weiterhin perorale flüssige Darreichungsformen von 2,4-Diamino-benzylpyrimidinen in Betracht gezogen, die dadurch gekennzeichnet sind, daß sie neben üblichen Hilfs- und Trägerstoffen ein Embonat von 2,4-Diamino-benzylpyrimidinen der allgemeinen Formel

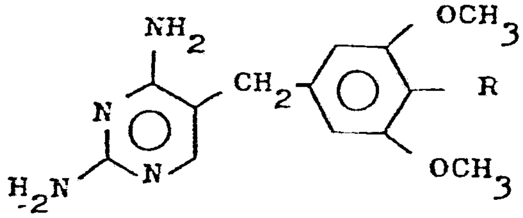 . 1/2 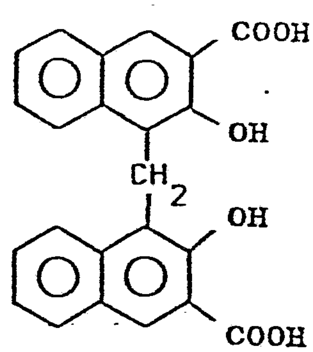

in der R für Methoxy oder Methoxyäthoxy steht, in mikronisierter Form, gegebenenfalls zusammen mit einem Sulfonamid, enthalten.

Durch die Erfindung wird schließlich weiterhin ein Verfahren zur Herstellung von peroralen flüssigen Darreichungsformen von 2,4-Diamino-benzylpyrimidinen in Betracht gezogen, das dadurch gekennzeichnet ist, daß man ein Embonat von 2,4-Diamino-benzylpyrimidinen der allgemeinen Formel

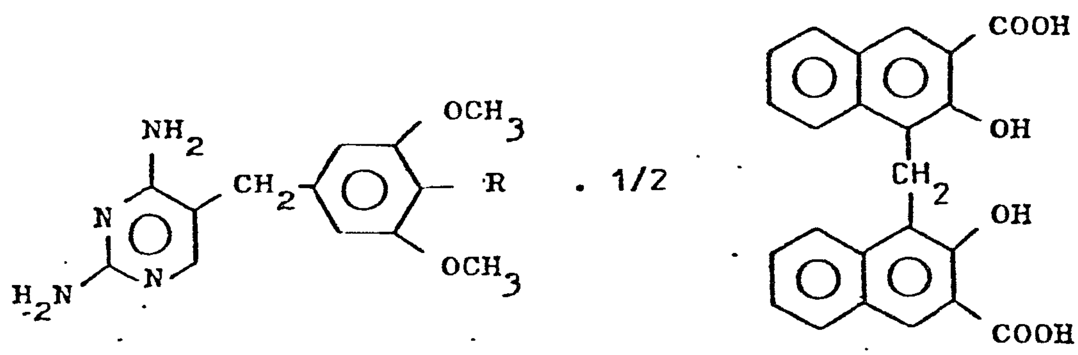

in der R für Methoxy oder Methoxyäthoxy steht, in mikronisierter Form, gegebenenfalls zusammen mit einem Sulfonamid, mit üblichen Hilfs- und Trägerstoffen vermischt.

Erfindungsgemäß werden die Embonate der oben angegebenen 2,4-Diamino-benzylpyrimidine, d.h. von Trimethoprim und Tetroxoprim in mikronisierter Form, vorzugsweise mit einer Teilchengröße ≦ 40 µm, besonders bevorzugt ≦ 10 µm, verwendet.

Die unter Verwendung der Embonate der obengenannten 2,4-Diamino-benzylpyrimidine hergestellten flüssigen Darreichungsformen können gegebenenfalls noch für derartige Kombinationspräparate übliche Sulfonamide, wie z.B. Sulfadiazin, enthalten. Die Teilchengröße des Sulfonamids ist vorzugsweise ≦ 200 µm.

Die Herstellung der flüssigen Zubereitungsformen unter Verwendung der Embonate der genannten 2,4-Diamino-benzylpyrimidine und gegebenenfalls von Sulfonamiden erfolgt in

an sich bekannter Weise, wie sie beispielsweise im "Pharmazeutische Technologie, Georg Thieme Verlag, Stuttgart, 1978" und "The Theory and Practice of Industrial Pharmacy, Second Edition, Lea & Febiger, Philadelphia, 1976" beschrieben wird. Zweckmäßigerweise legt man in einer Prozeßanlage mit Rührwerk und üblichen Einrichtungen den Träger, beispielsweise Zuckersirup DAB 8, vor und fügt die anderen Bestandteile des Saftes unter Rühren bei. Das Embonat wird vorzugsweise zuvor in einer Luftstrahlmühle auf die obengenannte Teilchengröße zermahlen. Wenn der Saft ein Sulfonamid enthält, dann wird dieses vorzugsweise nach dem Zermahlen auf die obengenannte Teilchengröße zugesetzt. Nach Zugabe sämtlicher Bestandteile des Safts wird eine Homogenisierung durchgeführt.

Die erfindungsgemäß hergestellten flüssigen Zubereitungen enthalten im allgemeinen 10 bis 100 mg/ml, vorzugsweise 15 bis 40 mg/l, 2,4-Diamino-benzylpyrimidin-Embonat und 25 bis 250 mg/ml, vorzugsweise 75 bis 200 mg/ml, Sulfonamid.

Die erfindungsgemäß hergestellten flüssigen Darreichungsformen, beispielsweise Säfte, zeichnen sich auch ohne Zusatz eines Geschmackskorrigens durch einen angenehmen nicht-bitteren Geschmack aus. Ein bitterer Geschmack ist im Gegensatz zu den bekannten Darreichungsformen, die das 2,4-Diamino-benzylpyrimidin in Form anderer Salze und der freien Base enthalten, bei den erfindungsgemäß hergestellten Präparaten nicht festzustellen.

Die Tagesdosis kann in einer oder mehreren Einzeldosen verabreicht werden. Neben den erfindungsgemäß verwendeten Embonaten von 2,4-Diamino-benzylpyrimidinen und gegebenenfalls Sulfonamiden enthalten die flüssigen Zubereitungsformen übliche Träger- und Hilfsstoffe, wie sie z.B. in den obengenannten Standardwerken der pharmazeutischen Technologie beschrieben werden.

Die Erfindung wird in den Beispielen erläutert.

Beispiel 1: Trimethoprim-Embonat

Eine Mischung aus 29 g (0,1 Mol) Trimethoprim und 19,5 g (0,05 Mol) Embonsäure wird unter lebhaftem Rühren in ein siedendes Gemisch aus 1000 ml Isopropanol und 500 ml Wasser eingetragen. Die nach wenigen Minuten erhaltene klare Embonat-Lösung läßt man zunächst bei Raumtemperatur und anschließend im Eisbad erkalten. Das auskristallisierte Embonat wird auf einer Fritte gesammelt, zunächst mit wenig Aceton, dann mit Äther/Pentan gewaschen und im Vakuum bei 60°C getrocknet (Ausbeute 45 g). Schwach gelb gefärbte, in Wasser, Alkohol und Aceton schwerlösliche Kristalle vom Fp 202°C.

Analyse: $C_{51}H_{52}N_8O_{12}$ (969,02)
Ber. C 63,23 H 5,41 N 11,56
Gef. C 63,38 H 5,58 N 11,30.

IR (KBr):3500-2500 $cm^{-1}$ (freies und gebundenes OH und NH); 1660, 1450 $cm^{-1}$ ($COO^-$ asym. und sym.)
$^1$H-NMR (DMSO-$d_6$): 3,6-3,9 ppm (s, 22H); 4,8 ppm (s, 2H); 6,6 ppm (s, 4H), 7,0-8,4 ppm (m, 24H).

Beispiel 2: Tetroxoprim-Embonat

Eine Mischung aus 100,3 g(0,3 Mol) Tetroxoprim und 58,3 g (0,15 Mol) Embonsäure wird unter lebhaftem Rühren in ein siedendes Gemisch aus 1200 ml Aceton und 600 ml Wasser

eingetragen. Die nach wenigen Minuten erhaltene klare Embonat-Lösung läßt man bei Raumtemperatur erkalten und saugt vom auskristallisierten Embonat ab. Dieses wird mit Aceton, dann mit Äther/Pentan gewaschen und im Vakuum bei 60°C getrocknet (Ausbeute 135 bis 150 g). Umkristallisieren in Aceton/Wasser. Schwach gelb gefärbte, in Wasser, Äthanol, Methanol und Aceton schwerlösliche Kristalle vom Fp 162 bis 170°C.

Analyse: $C_{55}H_{60}N_8O_{14}$ (1057,12)
Ber. C 62,49 H 5,72 N 10,60
Gef. C 62,33 H 5,75 N 10,56.

UV ($CH_3OH$) 365 nm (Embonat-Anion), 298 nm, 286 nm, 276 nm (Kation)

IR (KBr): 3560-2500 $cm^{-1}$ (freies und gebundenes OH und NH); 1655, 1445 $cm^{-1}$ ($COO^-$ asym. und sym.).

$^1$H-NMR (DMSO-$d_6$): 3,25 ppm (s, 6H7, 3,5-3,7 ppm (s, m, 8H), 3,75 ppm (s, 12H), 4,05 ppm (m, 4H), 5,1 ppm (s, 2H), 6,7 ppm (s, 4H), 4,9-8,6 ppm (m, 24H).

Beispiel 3: Herstellung eines Trimethoprim-Embonat enthaltenden Saftes

Es wird ein Saft mit folgender Zusammensetzung hergestellt:

| | |
|---|---|
| Trimethoprim-Embonat, mikronisiert | 267,0 g |
| p-Hydroxybenzoesäureester | 10,0 g |
| Natriumbenzoat | 25,0 g |
| Siliciumdioxid (kolloidal nach DAC/Aerosil 200) | 150,0 g |
| Polyvinylpyrrolidon (Kollidon 25) | 25,0 g |
| Zuckersirup nach DAB 8 | auf 10000 ml |

Das Trimethoprim-Embonat wird mit einer Luftstrahlmühle so vermahlen, daß der Rückstand auf dem 10 µm-Sieb nicht größer als 7% und der Rückstand auf dem 40 µm-Sieb nicht größer als 2% ist. Bei mikroskopischer Beurteilung sollte der Hauptanteil der Trimethoprim-Embonatteilchen bei 2 µm liegen.

In einer Prozeßanlage mit Rührwerk, Dissolver und Zahnkolloidmühle wird der Zuckersirup DAB 8 nach Arzneibuchvorschrift (DAB 8, S. 542-543) hergestellt. Nach Abkühlung auf ca. 50°C werden die p-Hydroxybenzoesäureester und das Natriumbenzoat hinzugefügt und so lange gerührt, bis eine klare Lösung entstanden ist.

Dann werden über die Eintragevorrichtung das Siliciumdioxid DAC und Polyvinylpyrrolidon eingetragen und gut verteilt. Anschließend wird das mikronisierte Trimethoprim-Embonat hinzugefügt. Hierauf wird 20 Minuten unter den obengenannten Bedingungen homogenisiert, wobei durch Kühlung die Temperatur auf 20°C begrenzt wird.

Der pH-Wert des Saftes liegt zwischen pH 5 bis 7, vorzugsweise zwischen pH 5,5 bis 6,5.

Beispiel 4: Herstellung eines Tetroxoprim-Embonat enthaltenden Saftes

Es wird ein Saft in folgender Zusammensetzung hergestellt:

| | |
|---|---|
| Tetroxoprim-Embonat, mikronisiert | 316,1 g |
| p-Hydroxybenzoesäureester | 10,0 g |
| Natriumbenzoat | 25,0 g |

| | |
|---|---|
| Siliciumdioxid (kolloidal nach DAC/Aerosil 200) | 150,0 g |
| Polyvinylpyrrolidon (Kollidon 25) | 25,0 g |
| Zuckersirup nach DAB 8 | auf 10000 ml |

Das Tetroxoprim-Embonat wird mit einer Luftstrahlmühle so vermahlen, daß der Rückstand auf dem 10 µm-Sieb nicht größer als 7% und der Rückstand auf dem 40 µm-Sieb nicht größer als 2% ist. Bei mikroskopischer Beurteilung sollte der Hauptanteil der Tetroxoprim-Embonatteilchen bei 2 µm liegen.

In einer Prozeßanlage mit Rührwerk, Dissolver und Zahnkolloidmühle wird der Zuckersirup DAB 8 nach Arzneibuchvorschrift (DAB 8, S. 542-543) hergestellt. Nach Abkühlung auf ca. 50°C werden die p-Hydroxybenzoesäureester und das Natriumbenzoat hinzugefügt und so lange gerührt, bis eine klare Lösung entstanden ist.

Dann werden das Siliciumdioxid DAC und Polyvinylpyrrolidon eingetragen und gut verteilt. Anschließend wird das mikronisierte Tetroxoprim-Embonat hinzugefügt. Hierauf wird 20 Minuten unter den obengenannten Bedingungen homogenisiert, wobei durch Kühlung die Temperatur auf 20°C begrenzt wird.

Der pH-Wert des Saftes liegt zwischen pH 5 bis 7, vorzugsweise zwischen pH 5,5 bis 6,5.

Beispiel 5: Herstellung eines Tetroxoprim-Embonat und Sulfadiazin enthaltenden Saftes

Es wird ein Saft in folgender Zusammensetzung hergestellt:

| | |
|---|---|
| Tetroxoprim-Embonat, mikronisiert | 316,1 g |
| Sulfadiazin BP | 500,0 g |
| p-Hydroxybenzoesäureester | 10,0 g |
| Natriumbenzoat | 25,0 g |
| Siliciumdioxid (kolloidal nach DAC/Aerosil 200) | 150,0 g |
| Polyvinylpyrrolidon (Kollidon 25) | 40,0 g |
| Zuckersirup nach DAB 8 | auf 10000 ml |

Das Tetroxoprim-Embonat wird mit einer Luftstrahlmühle so vermahlen, daß der Rückstand auf dem 10 µm-Sieb nicht größer als 7% und der Rückstand auf dem 40 µm-Sieb nicht größer als 2% ist. Bei mikroskopischer Beurteilung sollte der Hauptanteil der Tetroxoprim-Embonatteilchen bei 2 µm liegen.

In einer Prozeßanlage mit Rührwerk, Dissolver und Zahnkolloidmühle wird der Zuckersirup DAB 8 nach Arzneibuchvorschrift (DAB 8, S.542-543) hergestellt. Nach Abkühlung auf ca. 50°C werden die p-Hydroxybenzoesäureester und das Natriumbenzoat hinzugefügt und so lange gerührt, bis eine klare Lösung entstanden ist.

Dann werden das Siliciumdioxid DAC und Polyvinylpyrrolidon eingetragen und gut verteilt. Anschließend wird das mikronisierte Tetroxoprim-Embonat und das Sulfadiazin BP hinzugefügt. Hierauf wird 20 Minuten unter den obengenannten Bedingungen homogenisiert, wobei durch Kühlung die Temperatur auf 20°C begrenzt wird.

Der pH-Wert des Saftes liegt zwischen pH 5 bis 7, vorzugsweise zwischen pH 5,5 bis 6,5.

Ende der Beschreibung.

## Patentansprüche

1. Verwendung von Embonaten von 2,4-Diamino-benzyl-pyrimidinen der allgemeinen Formel

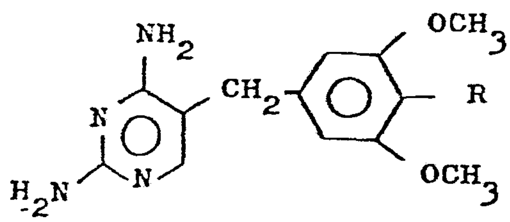 . 1/2 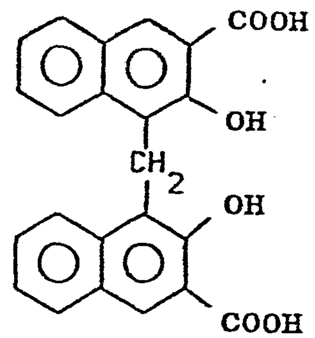

in der R für Methoxy oder Methoxyäthoxy steht, in mikronisierter Form zur Herstellung von peroralen flüssigen Darreichungsformen, die 2,4-Diamino-benzylpyrimidin gegebenenfalls zusammen mit einem Sulfonamid enthalten.

2. Verwendung von Embonaten von 2,4-Diamino-benzylpyrimidinen nach Anspruch 1, dadurch gekennzeichnet, daß die perorale flüssige Darreichungsform ein Saft, vorzugsweise zur Verwendung in der Kinderheilkunde, ist.

3. Verwendung von Embonaten von 2,4-Diamino-benzylpyrimidinen nach Anspruch 2, dadurch gekennzeichnet, daß das 2,4-Diamino-benzylpyrimidin Trimethoprim ist.

4. Verwendung von Embonaten von 2,4-Diamino-benzylpyrimidinen nach Anspruch 1 oder 2, dadurch ge-

k e n n z e i c h n e t , daß das 2,4-Diamino-benzylpyrimidin Tetroxoprim ist.

Europäisches Patentamt

**EUROPÄISCHER RECHERCHENBERICHT**

0048298

Nummer der Anmeldung

EP 80 10 5712

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| X | CHEMICAL ABSTRACTS, Band 89, 1978, Seite 538, Nr. 117802s Columbus, Ohio, U.S.A.<br>& ES - 452 885 (LABORATORIO MARTIN CUATRECASAS S.A.) 16-10-1977<br>* Zusammenfassung *<br>-- | 1-3 | A 61 K 31/505<br>31/63<br>31/635 |
| | BE - A - 850 975 (DR. ANDREV, BARCELONA)<br>* Seiten 2,3,9,10 *<br>---- | 1 | |

RECHERCHIERTE SACHGEBIETE (Int Cl )

C 07 D 239/48
A 61 K 31/505
31/635

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Grunden angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 07-05-1981 | FRANCOIS |